# EUROPEAN PATENT SPECIFICATION

(11) **EP 1 452 104 B1**
(45) Date of publication and mention of the grant of the patent: **20.04.2011**
(21) Application number: 03075613.4
(22) Date of filing: 28.02.2003
(51) Int. Cl.: A43D 25/18

(54) **Method of producing an absorbent article and an absorbent article produced according to the method**
Absorbierender Artikel und Verfahren zu deren Herstellung
Procede de fabrication de produit absorbant et produit ainsi obtenu

(43) Date of publication of application: 01.09.2004
(73) Proprietor: SCA Hygiene Products AB, 405 03 Göteborg (SE)
(72) Inventor: Runeman, Bo, 433 75 Jonsered (SE); Farbrot, Anne, 436 39 Askim (SE); Hjorth, Madeleine, 413 20 Göteborg (SE); Dovertie, Ralph, 426 53 Västra Frölunda (SE)
(74) Representative: Andersson, Per Rune

(56) References cited:
- EP-A- 1 250 940
- WO-A-00/48544
- WO-A-00/69484
- US-A- 4 785 996
- US-A- 5 643 588
- US-A- 6 149 934

## Description

### TECHNICAL FIELD:

The invention relates to a method for applying a lotion to a surface of an absorbent article, said method comprising the steps of: placing the surface of the absorbent article in the proximity of a spraying apparatus comprising a spray head having an orifice and pushing the lotion through the spray head orifice.

### BACKGROUND ART:

Absorbent articles having lotion applied on e.g. the topsheet have been known within the area for quite some time now. By applying lotion on an absorbent article such as a diaper, an incontinence product, a sanitary napkin or the like, added value is given to the product.

As described in WO 96/16682, the lotion, or similar, could be placed on the topsheet of a diaper. In this manner, the lotion is said to add barrier properties to the skin, reducing the irritation caused by contact with faeces.

Further back in time, similar use of lotions was known e.g. through US 3,896,807, which describes how the surface of a diaper can be furnished with an emulsifiable phase which upon exposure to moisture will form a water/oil (W/O) or oil/water (O/W) emulsion. During use of the product, the emulsion will transfer to the user's skin and will act as a vehicle for adding skin caring substances to the user's skin.

In earlier published literature, several examples are given of how to apply the lotion on absorbent articles.

In WO 96/16681 spraying, printing (e.g. flexographic printing), coating and extrusion are mentioned as examples of application methods. With the spray technique, it is said to be possible to spray patterns having a dimension of 9x18 cm.

In WO 98/24390 and WO 99/45974, slot coating, extrusion coating, gravure coating, spraying and printing are mentioned. The lotion can be applied in stripes, squares, dots or spirals.

WO 00/48544 describes a bit more thoroughly the techniques used to apply lotion on absorbent articles. More specifically, spraying is described. When spraying, the lotion is propelled through a nozzle by means of air. However, it can also be propelled through the nozzle substantially without the use of air. Further, it is described that the nozzle can be mechanically manipulated during spraying to form patterns such as spirals, squares, stripes or circles. In order to achieve a desired pattern such as a square, the nozzle itself needs to be altered. It is also stated that the stripes have a minimum with of 0.1 inches.

The existing spray technique offers some advantages to the producer of absorbent articles wanting to give added values to absorbent articles. However, several disadvantages still exist that prior art has failed to remove.

When the lotion is sprayed through the orifice of the nozzle at high speed, it is subjected to a high amount of stress. The stress is not only purely mechanical stress, but is also caused by pressure differences. This causes the lotion to turn into an aerosol fully or partly, something that greatly affects the quality of the applied pattern and the flexibility of the process.

Even if it is possible to make patterns such as spirals, stripes or circles, the applied pattern becomes diffuse in the periphery and the aerosolised spray string makes it difficult to add lotion in an exact manner.

The nozzle of the spray head needs to be in close proximity to the sprayed surface. This prevents the spray string from being spread too much in an uncontrolled manner. However, this is a severe limitation to process flexibility since even if the slightest amount of aerosol is formed, it will complicate the handling and the flexibility of the process.

The fine droplets from the aerosol which lie in the periphery of the sprayed string can easily be sucked down into the absorbent core by the capillary forces of the absorbent core or an acquisition layer. This reduces the absorbent capacity and the acquisition rate of the absorbent article. Accordingly, it is important to keep the lotion on the surface of the absorbent article.

From a process point of view, it is difficult to handle aerosols when manufacturing absorbent articles. Reducing the amount of aerosol vastly reduces the required amount of cleaning and maintenance of the production equipment.

Consequently, there is a need for a method of applying lotion to absorbent articles in a simple and efficient manner. Hence, it is an object of the current invention to solve the above-mentioned problems and to provide an improved method for applying lotion to absorbent articles. It is also an object of the invention to provide an absorbent article having lotion arranged in a well-defined application pattern.

### DISCLOSURE OF INVENTION:

The current invention provides a method of applying lotion to an absorbent article. The method of the invention is distinguished in that the lotion is sprayed as a continuous fiberised string.

The term "fiberised string" means that the lotion is in the form of a continuous string and that no amount of aerosol is formed. In contrast to ordinary lotion which is sprayed in a continuous pattern, the lotion sprayed as a fiberised string forms no aerosol or droplets when leaving the spray head orifice. The lotion can be laid in an intermittent pattern or in a continuous pattern. However, the string in itself is still continuous. In addition, the fiberised string is relatively thin as compared to ordinary sprayed strings.

More specifically, the invention concerns a method for applying a lotion to a surface of an absorbent article, said method comprising the steps of: placing the surface of the absorbent article in the proximity of a spraying apparatus comprising a spray head having an orifice; pushing the lotion through the spray head orifice and propelling the lotion to the surface as a continuous fiberised string.

The term "proximity" should not be taken to mean that the spraying apparatus needs to be in direct contact or even close to the surface of the absorbent article. In accordance with the invention, the spray head and the spraying apparatus can be separated by several meters of hose or pipe or similar. The term proximity should be interpreted as indicating a correlating relationship between the surface of the absorbent article, the spray head and the spraying apparatus.

By propelling the lotion as a continuous fiberised string instead of spraying in stripes or ordinary lines, several advantages are obtained. Due to the use of a fiberised string, the spray head can be placed within a wide variety of distances from the surface of the absorbent article. In one embodiment of the invention the surface is placed at a distance of 15-300 mm from the spray head.

Several different kinds of spray heads can be used. In one embodiment of the invention the lotion is propelled from the orifice by an air stream that is separated from the orifice. In another embodiment of the invention the lotion is sprayed with a velocity of at least 80 m/min.

In a preferred embodiment of the invention the lotion is propelled to the surface of the absorbent article as a spiral continuous fiberised string.

The invention makes it possible to spray very thin fiberised strings of lotion. This saves lotion as well as prohibits blocking of e.g. the topsheet of the absorbent article. In one embodiment of the invention the fiberised string has a diameter of less than 2.2 mm, preferably less than 1 mm and most preferably less than 0.75 mm.

Surprisingly, the inventor has found that by using spray equipment normally used for spraying hot-melt adhesive and a lotion with an elastic behaviour measured by storage modulus G'>0.2Pa measured at 55°C, preferably a storage modulus G'>0.6Pa measured at 55°C, remarkable results are achieved.

Using lotion with a storage modulus G'>0.2Pa measured at 55°C combined with this technique offers the possibility of processing the lotion at high temperatures without lotion disruption or the formation of lotion aerosols or droplets. This prohibits the lotion from forming lumps due to processing in temperatures close to the lotion melting point. Such lumps could otherwise cause blockage of tubes or nozzles.

The technique also makes it possible to obtain a cleaner environment around the process. By eliminating the formation of aerosols due to lotion disruption, less cleaning and less maintenance is required.

The spray technique in accordance with the invention and the specified lotion parameters make it possible to offer a more flexible production than with earlier spray techniques or techniques such as slot-coating. Moreover, the invention makes it possible to spray from a longer distance than before and to apply more exact patterns on the surface of the absorbent article. As an example, it is now possible to spray the lotion in three-dimensional-like patterns, stacking several strings on each other and forming a web-like lotion pattern.

Due to its elastic behaviour, the lotion is prevented from being sucked into the absorption core by capillary action. Instead, the lotion stays on the topsheet of the absorbent article.

In one embodiment of the invention, a spray head, is used to apply lotion on an absorbent article, the spray head comprising an orifice where said lotion passes during use. Several air stream outlets are placed around and separated from the orifice so that, during use, air streams propel the lotion in a continuous fiberised string away from the orifice and the spray head.

The spray head is used in combination with a spraying apparatus, to apply lotion on an absorbent article.

The lotion is pushed through the orifice of the spray head and the nozzle and then propelled by separated air streams against the surface of the absorbent article. The air streams are arranged symmetrically around the orifice of the nozzle. The air streams are separated from the orifice, which means that the lotion and the air do not leave the nozzle from the same orifice.

The lotion can be applied to the topsheet of the absorbent article, to cuffs or to any other surface or surfaces of the absorbent article.

In accordance with the invention, it is also possible to form very thin fiberised strings. The fiberised strings can have a diameter of less than 2.2 mm, preferably of less than 1 mm and most preferably of less than 0.75 mm.

When compared to conventional application patterns with strips of a width dimension in the order of 2.5-5 mm, the small fiberised strings give the opportunity to add lotion to the topsheet of the absorbent article without risking blocking the topsheet. An application pattern coating a large percentage of the topsheet area will reduce the acquisition rate of the absorbent article and indirectly the absorption capacity.

The invention also relates to an absorbent article manufactured according to said method and comprising a substantially liquid impermeable backsheet, a substantially liquid permeable topsheet and an absorbent core placed between the backsheet and the topsheet. The absorbent article further comprises lotion. The lotion is in the shape of a continuous fiberised string and exhibits a storage modulus of at least 0.2 Pa measured at 55°C.

In another embodiment of the invention, the lotion exhibits a storage modulus of at least 0.6 Pa measured at 55°C. In yet another embodiment of the invention the lotion exhibits a loss modulus of at least 2.0 Pa measured at 55°C.

It is well within the scope of the invention to use different kinds of lotion. However, it is important that the storage modulus is at least 0.2 Pa measured at 55°C.

The lotion composition used in the invention can be chosen from;
5 to 50% by weight of a component melting in the range from 25°C to 37°C, chosen from the group of paraffins, fatty acid esters, polyhydroxy fatty acid esters, fatty alcohols, alkoxylated fatty acid esters, alkoxylated fatty alcohols and mixtures of these compounds and
5 to 50% by weight of a component melting in the range from 40°C to 60°C, chosen from the group of polyhydroxy fatty acid esters, C14-C22-fatty alcohols, C12-C22-fatty acids, the alkoxylated derivatives of the fatty alcohols and fatty esters, and mixtures of these components, and
25% to 45% by weight of water.

In another embodiment of the invention, the lotion is placed on the topsheet of the absorbent article as a continuous fiberised string.

### BRIEF DESCRIPTION OF DRAWINGS:

The invention will be described in greater detail below with reference to the figures shown in the accompanying drawings, in which:
- Fig. 1a and 1b: show a spray head,
- Fig. 2: shows a spray gun comprising a spray head,
- Fig. 2a,: shows a spray apparatus,
- Fig. 3: shows a cross-section of a nozzle when lotion is pushed through the orifice,
- Fig. 4: shows several spray guns/modules attached in a side-by-side relationship,
- Figs. 5-10: show different spray patterns; and
- Fig. 11-12: show temperature sweep graphs.

### DETAILED DESCRIPTION:

The invention can be applied to several different kinds of absorbent articles such as pant diapers, diapers, incontinence devices, sanitary napkins, pantiliners, micro-sanitary napkins etc.

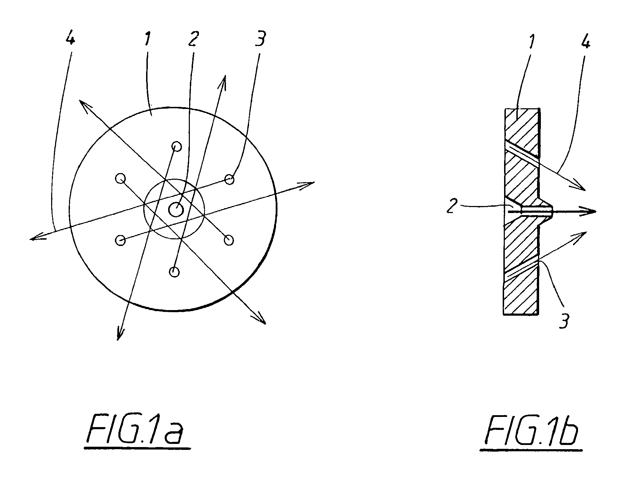

Figs. 1a and 1b show a spray head 1 for the application of lotion to an absorbent article. The spray head 1 has a circular cross-section and is provided with an orifice 2, which also has a circular cross-section. The orifice 2 is centrally placed on the spray head 1. Separate air stream orifices 3 are symmetrically placed around the central orifice 2, and are arranged to pass streams of air 4 in specified directions during use of the spray head.

The directed air streams 4 propel lotion towards the surface of an absorbent article, forming a fiberised string of lotion 5 in the shape of a helix. The fiberised string of lotion does not penetrate the topsheet and is prevented from decreasing the absorption capacity of the absorbent article.

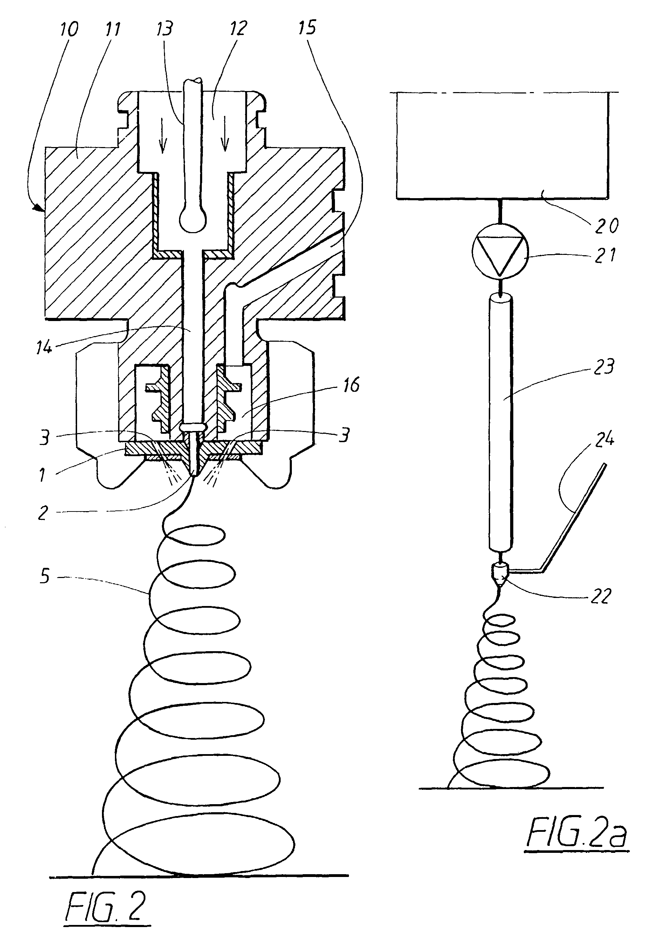

Fig 2 shows a schematic representation of the spray head 1 mounted in a part of a spray gun 10. The spray gun 10 comprises a cover 11 which comprises a temporary container 12 for the lotion. In the container 12 there is a temperature regulator 13 with which the temperature of the lotion can be regulated. From the temporary container 12, a small pipe 14 connects the central orifice 2 with the temporary container 12 on the spray head 1.

An air channel 15 supplies air to a chamber 16 arranged within the cover 11, the chamber 16 being in direct connection with the air stream orifices 3 on the spray head 1. The chamber 16 is provided with an air stream regulator by which it is possible to regulate the flow of the air streams.

Fig. 2a shows a schematic representation of a spraying apparatus such as is used in the invention. The apparatus comprises a lotion storage container 20 and a pump 21 for pumping the lotion from the storage container 20 to a spray head 22, the spray head 22 and the storage pump 21 being connected by a heated hose 23. Pressurised air 24 is supplied to the spray head 22.

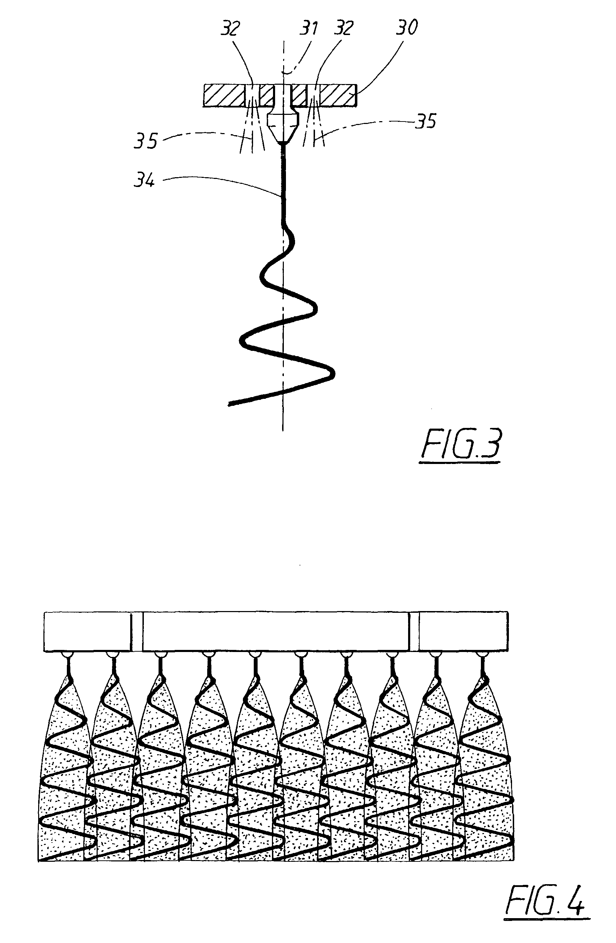

Fig. 3 shows a cross-section of a nozzle 30. The nozzle 30 has a centrally placed orifice 31 and two separated air stream orifices 32, placed close to the central orifice 31 on either side thereof. Fig. 3 also shows how lotion is pushed trough the central orifice 31 and is propelled by air streams 35 as it leaves the central orifice 31.

Fig. 4 shows how several spray guns or modules are placed next to each other in order to produce a broader pattern of lotion on the sprayed surface.

Spray heads of this type are commercially available for hot-melt adhesive applications under the trademark Controlled Fiberization Spray Nozzles, manufactured by Nordson® Corporation, 2905 Pacific Drive, Norcross, Georgia 30071, United States.

Additional models are also available from Nordson®. The spraying technique is described further in US 4,785,996, US 4,815,660 and in EP 474,155.

### The lotion composition:

The lotion preferably is a composition comprising at least 5 to 50% by weight of a component a) melting in the range from 25°C to 37°C, chosen from the group of paraffins, fatty acid esters, polyhydroxy fatty acid esters, fatty alcohols, alkoxylated fatty acid esters, alkoxylated fatty alcohols and mixtures of these compounds, and 5 to 50% by weight of a component b) melting in the range from 40°C to 60°C, chosen from the group of polyhydroxy fatty acid esters, C₁₄-C₂₂-fatty alcohols, C₁₂-C₂₂-fatty acids, the alkoxylated derivatives of the fatty alcohols and fatty esters, and mixtures of these components, and c) 25% to 45% by weight of water.

The compositions according to the invention are emulsions or suspensions. The emulsions are preferably of the O/W or W/O type.

By choosing the composition so that the storage modulus exceeds 0.2 Pa, a continuous fiberised string can be achieved when spraying the lotion.

In addition to specific emulsifiers and selected oily substances, the emulsions comprise 25-45% by weight, preferably 25-40% by weight and in particular 25-35% by weight, of water. The compositions according to the invention are preferably in the form of W/O emulsions.

Component a) can be chosen from a large number of compounds known to the person skilled in the art, the melting point should be in the range from 25°C to at most 37°C. Firstly, for this purpose it is possible to use certain paraffins, but also fatty acid esters and, in particular, fatty alcohols. Suitable paraffins are preferably semisolid paraffins, such as soft paraffin, preferably petrolatum. Suitable fatty alcohols are, for example, dodecanol or ricinol alcohol, to name one representative of the unsaturated fatty alcohols. The use of glycerides is particularly suitable, here preferably mixtures of partial glycerides and triglycerides, which should have the desired melting point of from 25°C to 37°C. Particular preference is given here to mixtures of glycerides of fatty acids having 8 to 18 carbon atoms.

Glycerides represent mono-, di- and/or triesters of glycerol with fatty acids, namely, for example, caproic acid, caprylic acid, 2-ethylhexanoic acid, capric acid, lauric acid, isotridecanoic acid, myristic acid, palmitic acid, palmoleic acid, stearic acid, isostearic acid, oleic acid, elaidic acid, petroselic acid, linoleic acid, linolenic acid, elaeostearic acid, arachidic acid, gadoleic acid, behenic acid and erucic acid, and technical-grade mixtures thereof. They conform to the formula (I), 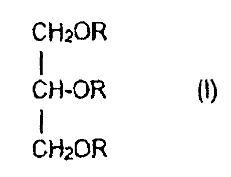 in which R is a COR' radical, in which R' is a branched or unbranched, saturated or unsaturated alkyl radical having 6 to 22 carbon atoms, and/or independently thereof, is hydrogen. Typical examples are lauric acid monoglyceride, lauric acid diglyceride, coconut fatty acid monoglyceride, coconut fatty acid triglyceride, palmitic acid monoglyceride, palmitic acid triglyceride, stearic acid monoglyceride, stearic acid diglyceride, isostearic acid monoglyceride, isostearic acid diglyceride, oleic acid monoglyceride, oleic acid diglyceride, tallow fatty acid monoglyceride, tallow fatty acid diglyceride, behenic acid monoglyceride, behenic acid diglyceride, erucic acid monoglyceride, erucic acid diglyceride, and technical-grade mixtures thereof, which may also comprise small amounts of triglyceride as secondary components from the preparation process.

An essential feature of the lotion is the emulsifier component c). Particularly suitable are glycerol partial esters with C₁₂-C₂₁ fatty acids, preferably glycerol monolaurate. Further, polyvinyl stearol ether and, particularly preferably, polyglycerol poly-12-hydroxystearate can be used.

Polyol poly-12-hydroxystearates are known substances which are sold, for example, under the names Dehymuls, PWPH or Eumulgin VL75 or Dehymuls SP11 by Cognis Deutschland GmbH. Further details regarding these compounds are given in European Patent Specification EP 0 766 661.

The polyol component of these compounds can be derived from substances which have at least 2, preferably 3 to 12 and in particular 3 to 8, hydroxyl groups and 2 to 12 carbon atoms. Typical examples are glycerol, polyglycerol, alkylene glycols, such as, for example, ethylene glycol, diethylene glycol and propylene glycol, methylol compounds, preferably trimethylolethane, trimethylolpropane, trimethylolbutane, pentaerythritol or dipentaerythritol, alkyl oligoglycosides having 1 to 22, preferably 1 to 8 and in particular 1 to 4, carbon atoms in the alkyl radical, sugar alcohols having 5 to 12 carbon atoms, for example sorbitol or manitol, and sugars having 5 to 12 carbon atoms, preferably glycose or sucrose, and also amino sugars, for example glucamine. The reaction product of poly-12-hydroxystearic acid with polyglycerol has proven particularly advantageous. Here, the polyglycerol has the following composition: glycerols 5 to 35% by weight, diglycerols 15 to 40% by weight, triglycerols 10 to 35% by weight, tetraglycerols 5 to 20% by weight, pentaglycerols 2 to 10% by weight and the remainder oligoglycerols.

In addition to the component a) and the component b) and water, the compositions according to the invention may also comprise further constituents, in particular further emulsifiers, preferably nonionic emulsifiers. Nonionic emulsifiers are characterized by their skin friendliness and mildness and their ecotoxicologically good properties. The use of a combination of nonionic emulsifiers gives particularly finely divided emulsions, meaning that the stability of the composition is increased. The composition according to the invention comprises the coemulsifiers in an amount of from 0 to 15% by weight, preferably from 1% to 10% by weight and in particular from 3% to 10% by weight, based on the total weight of the composition.

Furthermore, the compositions according to the invention may contain further conventional ingredients, for example silicone waxes or polysiloxanes, in amounts of from 1% to 6% by weight, preferably from 1.5% to 5.5% by weight and in particular from 2% to 5% by weight. Polysiloxanes are known polymeric compounds which contain the following structure as monomer units:

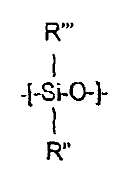

Here, R" and R"', independently of one another, are hydrogen or an alkyl, cycloalkyl, aryl or alkenyl radical. Siloxanes of this type preferably have viscosities at 37°C in the range from 5 to 5000 mPa s.

In addition, and preferably, the lotion may comprise skin-friendly or skincare substances, in amounts of from 0.1 % to 10% by weight, preferably from 1% to 8% by weight most preferably from 2 to 6% by weight. Ingredients of this type may, for example, be bisabolol, alantoin and panthenol. It is also possible to use vitamins, preferably vitamin E and vitamin precursors, and protein hydrolysates. Also suitable are plant extracts, such as from camomile, aloe vera, lime blossom, horse chestnut, green tea, oak bark, stinging nettle, hops, burdock, horsetail, hawthorn, almond, spruce needle, almond wood, juniper, coconut, apricot, lemon, wheat, kiwi, melon, orange, grapefruit, sage, rosemary, birch, mallow, thyme, balm, restharrow, coltsfoot, ginseng and root ginger. In addition, however, other skincare substances may also be present. Those which may be named here are, in particular, chitosan, and zinc oxide or zinc ricinoleate.

In a particular embodiment, the emulsions may comprise further optional additives, such as, for example, superfatting agents, thickeners, polymers, waxes, biogenic active ingredients, deodorant active ingredients, film formers, UV light protection factors, antioxidants, hydrotropic agents, preservatives, insect repellents, self-tanning agents, solubilizers, stabilizers, perfume oils, dyes, antimicrobial agents and the like.

Superfatting agents which can be used are substances such as, for example, lanolin and lecithin, and polyethoxylated or acylated lanolin and lecithin derivatives, polyol fatty acid esters, monoglycerides and fatty acid alkanolamides, the latter also serving as foam stabilizers.

Suitable thickeners are, for example, Aerosil grades (hydrophilic silicas), polysaccharides, in particular xanthan gum, guar guar, agar agar, alginates and Tyloses, carboxymethylcellulose and hydroxyethylcellulose, and also relatively high molecular weight polyethylene glycol mono- and diesters of fatty acids, polyacrylates (e.g. Carbopols® from Goodrich or Synthalens® from Sigma), polyacrylamides, polyvinyl alcohol and polyvinylpyrrolidone, surfactants, such as, for example, ethoxylated fatty acid glycerides, esters of fatty acids with polyols, such as, for example, pentaerythritol or trimethylolpropane, fatty alcohol ethoxylates with a narrowed homologue distribution or alkyl oligoglucosides, and electrolytes such as sodium chloride and ammonium chloride.

Suitable cationic polymers are, for example, cationic cellulose derivatives, such as, for example, a quaternized hydroxyethylcellulose available under the name Polymer JR 400® from Amerchol, cationic starch, copolymers of diallylammonium salts and acrylamides, quaternized vinylpyrrolidone/vinylimidazole polymers, such as, for example, Luviquat® (BASF), condensation products of polyglycols and amines, quaternized collagen polypeptides, such as, for example, lauryldimonium hydroxypropyl hydrolysed collagen (Lamequat®L/Grünau), quaternized wheat polypeptides, polyethyleneimine, cationic silicone polymers, such as, for example, amidomethicones, copolymers of adipic acid and dimethylaminohydroxypropyldiethylenetriamine (Cartaretins®/Sandoz), copolymers of acrylic acid with dimethyldiallylammonium chloride (Merquat® 550/Chemviron), polyaminopolyamides, cationic chitin derivatives, such as, for example, quaternized chitosan, optionally in microcrystalline dispersion, condensation products from dihaloalkyls, such as, for example, dibromobutane with bisdialkylamines, such as, for example, bis-dimethylamino-1,3-propane, cationic guar gum, such as, for example, Jaguar® CBS, Jaguar® C-17, Jaguar® C-16 from Celanese, quaternized ammonium salt polymers, such as, for example, Mirapol® A-15, Mirapol® AD-1, Mirapol® AZ-1 from Miranol.

Suitable anionic, zwitterionic, amphoteric and nonionic polymers are, for example, vinyl acetate/crotonic acid copolymers, vinylpyrrolidone/vinyl acrylate copolymers, vinyl acetate/butyl maleate/isobornyl acrylate copolymers, methyl vinyl ether/maleic anhydride copolymers and esters thereof, uncrosslinked polyacrylic acids, polyacrylic acids crosslinked with polyols, acrylamidopropyltrimethylammonium chloride/acrylate copolymers, octylacrylamide/methyl methacrylate/tert-butylaminoethyl methacrylate/2-hydroxypropyl methacrylate copolymers, polyvinylpyrrolidone, vinylpyrrolidone/vinyl acetate copolymers, vinylpyrrolidone/dimethylaminoethyl methacrylate/vinylcaprolactam terpolymers, and optionally derivatized cellulose ethers and silicones.

Biogenic active ingredients are to be understood as meaning, for example, tocopherol, tocopherol acetate, tocopherol palmitate, ascorbic acid, deoxyribonucleic acid, retinol, bisabolol, allantoin, phytantriol, panthenol, α-hydroxycarboxylic acids, amino acids, ceramides, pseudoceramides, essential oils, plant extracts and vitamin complexes.

Suitable deodorant active ingredients are, for example, antiperspirants, such as, for example, aluminium chlorhydrate, aluminium zirconium chlorhydrates, and zinc salts. These are used for the preparation of antiperspirant and deodorizing preparations and probably act via the partial closure of the sweat glands by protein and/or polysaccharide precipitation. In addition to the chlorhydrates, it is also possible to use aluminiumhydroxylactates and acidic aluminium/zirconium salts. An aluminium chlorhydrate which corresponds to the formula [Al₂(OH)₅Cl]·2.5 H₂O and whose use is particularly preferred is commercially available under the tradename Locron® by Clariant GmbH, for example. Likewise preferred according to the invention is the use of aluminium zirconium tetrachlorohydrex glycine complexes, which are marketed, for example, by Reheis under the name Rezal® 36G. Further deodorant active ingredients which may be added are esterase inhibitors. These are preferably trialkyl citrates, such as trimethyl citrate, tripropyl citrate, triisopropyl citrate, tributyl citrate and, in particular, triethyl citrate (Hydagen® C.A.T., Cognis Deutschland GmbH). The substances inhibit the enzyme activity, thus reducing the formation of odour. Presumably, in this process, the cleavage of the citric ester results in the release of the free acid, which lowers the pH on the skin sufficiently for the enzymes to be inhibited. Further substances which are suitable as esterase inhibitors are sterol sulphates or phosphates, such as, for example, the sulphates and phosphates of lanosterol, cholesterol, campesterol, stigmasterol and sitosterol, dicarboxylic acids and esters thereof, such as, for example, glutaric acid, monoethyl glutarate, diethyl glutarate, adipic acid, monoethyl adipate, diethyl adipate, malonic acid and diethyl malonate, hydroxycarboxylic acids and esters thereof, such as, for example, citric acid, malic acid, tartaric acid or diethyl tartrate. Antibacterial active ingredients which influence the bacterial flora and destroy bacteria which decompose perspiration or inhibit them in their growth can likewise be present in the emulsions. Examples thereof are chitosan, phenoxyethanol and chlorhexidine gluconate. 5-Chloro-2-(2,4-dichlorophenoxy)phenol, which is sold under the name Irgasan® by Ciba-Geigy, Basle/CH, have proven particularly effective.

To improve flow behaviour of the composition, it is also possible to use hydrotropic agents, such as, for example, ethanol, isopropyl alcohol, or polyols. Polyols that are suitable here preferably have 2 to 15 carbon atoms and at least two hydroxyl groups. The polyols can also contain further functional groups, in particular amino groups, or be modified with nitrogen. The overall fraction of optional additives may be from 1% to 50% by weight, preferably from 5% to 40% by weight, based on the compositions.

Furthermore, it may be advantageous to co-use optional components to stabilise the emulsion, for example glycerol or magnesium sulphate, preferably in amounts of in each case 0.1 to at most 5% by weight, preferably from 0.1 to 1.5% by weight.

The composition can be prepared by customary cold or hot processes; preference is given to using the phase inversion temperature method.

In the following examples the following composition was used:
84
   40% by weight of partial glyceride/triglyceride mixture based on C₁₄/C₁₆-fatty acids, melting range: 33.0°C -35.5°C
   15% by weight of partial glyceride/triglyceride mixture based on C₁₄/C₁₆-fatty acids, melting range 40.0°C -42.0°C
   13% by weight of polyvinyl stearyl ether
   1.45% by weight of glycerol (86% pure)
   0.30% by weight of MgSO₄ × 7 H₂O
   Remainder: water
58
   Reference lotion comprising:
   50-60% by weight of a mixture of partial glycerides of coconut fatty acids, melting point 30-34°C
   10-20% by weight of stearyl alcohol, melting point 56-60°C
   20% by weight of polyvinyl stearyl ether, melting point 45-48°C
   2% by weight of silicone wax.

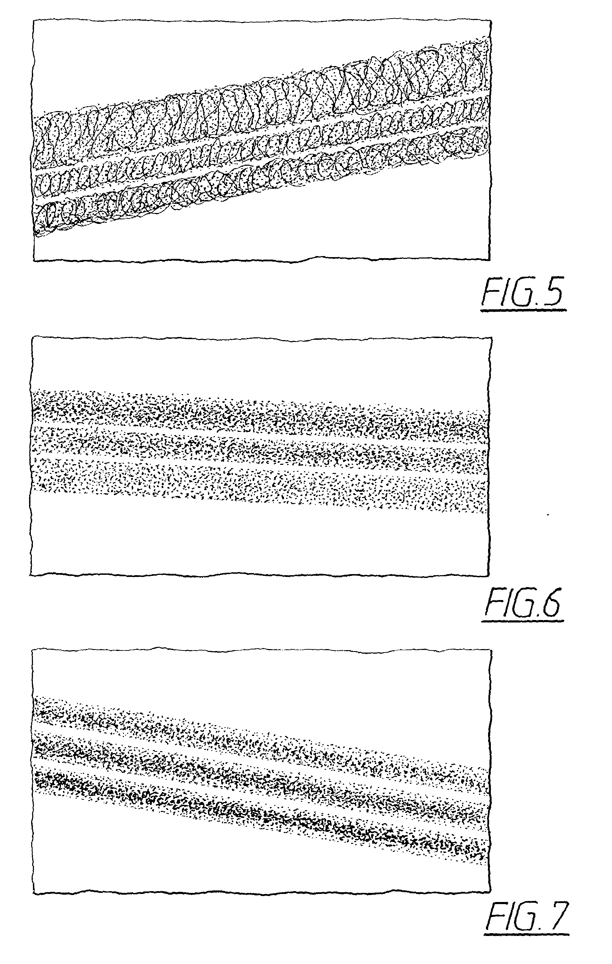

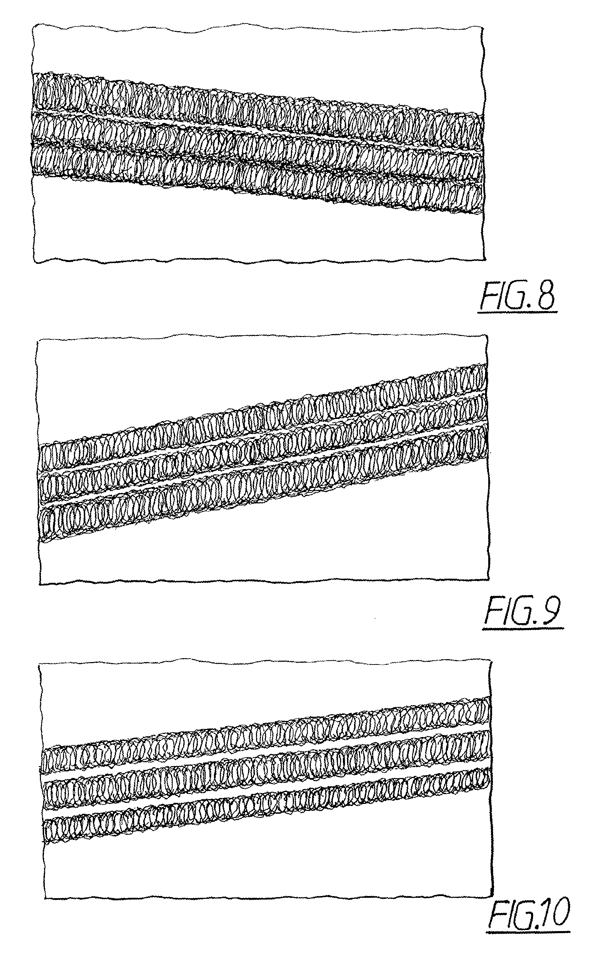

Results from Figs. 5-10, spray tests when spraying with equipment shown in fig. 2a with two different lotions at different temperatures.

| Sample | containerr (°C) | tube (°C) | Nozzle (°C) | Pattern when spraying |
|---|---|---|---|---|
| | | | | |
| 58(5) | 55 | 55 | 55 | Aerosol/traces of string pattern- fig. 5 |
| 58(6) | 60 | 60 | 60 | Aerosol pattern - fig. 6 |
| 58(7) | 60 | 65 | 70 | Aerosol pattern - fig. 7 |
| | | | | |
| 84 (8) | 55 | 55 | 55 | Continuous fiberised string |
| 84(10) | 60 | 60 | 60 | Continuous fiberised string |
| 84 (2) | 65 | 68 | 75 | Continuous fiberised string |

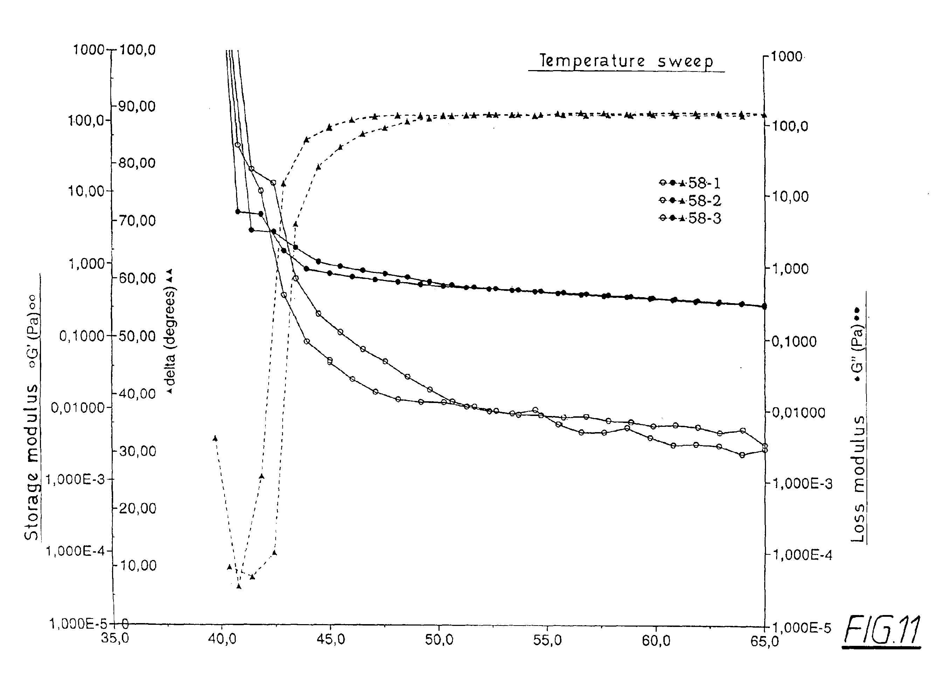

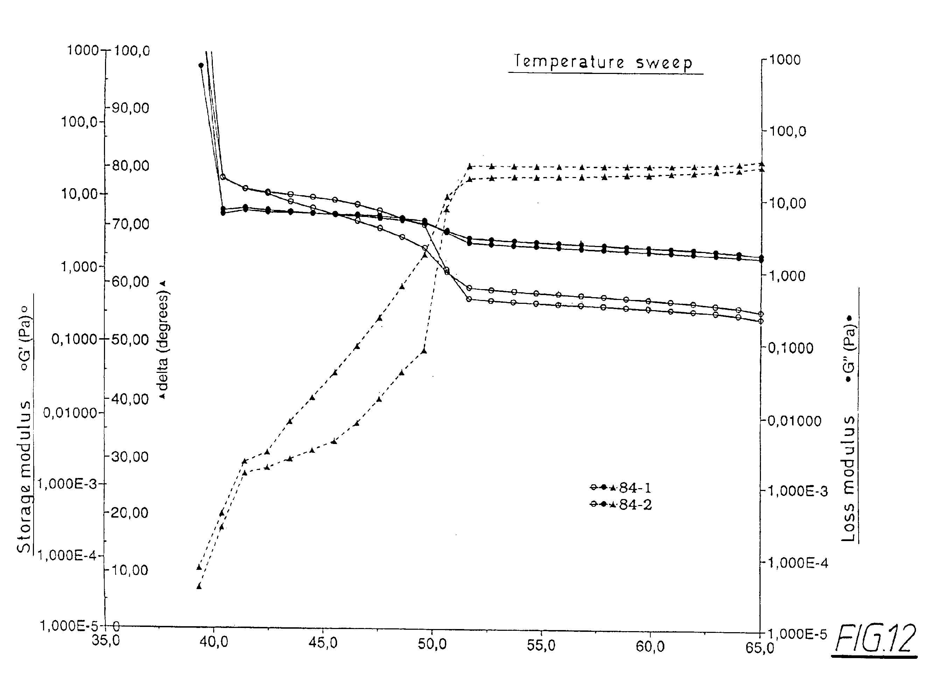

Characteristics at 55°C (from fig.11-12):

| | | |
|---|---|---|
| Lotion | 58 | 84 |
| Loss modulus | 0.3 Pa | 2.0 Pa |
| Storage modulus | 0.01 Pa | 0.6 Pa |

The measurements were made using a Rheometer AR 1000-N, TA Instruments, UK:
Temperature sweep:

| | |
|---|---|
| Oscillation procedure | Temperature sweep |
| Material | Acrylic plate φ 4cm |
| Gap | 200 µm |
| Temperature interval | 65-35°C |
| Torque | 10µNm |
| Frequency | 1Hz |

Hot melt adhesive equipment (such as described in fig. 2a) parameters when spraying with lotion 58:

| | |
|---|---|
| Module | Nordson, five modules (two blocked) |
| Spray head | 3 Nordson Nr. 755957-φ12 (0,012) -12 air jets-SPC |
| Container | Nordson with pump pr6m1. |
| Temp (sample5) | Container: 55°C, tube: 55°C, nozzle: 55°C |
| Temp (sample 6) | Container: 60°C, tube: 60°C, nozzle: 60°C |
| Temp (sample 7) | Container: 60°C, tube: 65°C, nozzle: 70°C |
| Speed | 80m/min |
| Spray air pressure | 0.4bar |
| Distance spray head/material | 15mm |

Hot melt adhesive equipment (such as described in fig. 2a) parameters when spraying with lotion 84:

| | |
|---|---|
| Module | Nordson, five modules (two blocked) |
| Spray head | 3 Nordson Nr. 755957-φ12 (0,012) -12 air jets-SPC |
| Container | Nordson with pump pr6m1. |
| Temp (sample 8) | Container: 55°C, tube: 55°C, nozzle: 55°C |
| Temp (sample 10) | Container: 60°C, tube: 60°C, nozzle: 60°C |
| Temp | Container: 65°C, tube: 68°C, nozzle: 75°C |
| Speed | 80m/min |
| Spray air pressure | 0.4bar |
| Distance spray head/material | 15mm |

## Claims

1. Method for applying lotion (5) to a surface of an absorbent article, said method comprising the steps of;
- placing the surface of the absorbent article in the proximity of a spraying apparatus comprising a spray head (1) having an orifice (2),
- pushing the lotion (5) through the spray head orifice (2),
**characterised in that**
the lotion (5) is propelled to the surface as a continuous fiberised string.

2. Method according to claim 1, **characterised in that** the surface of the absorbent article is placed at a distance of from 15 to 300 mm from the spray head (1).

3. Method according to claim 1, **characterised in that** the lotion is propelled from the orifice by an air stream (4) which is separated from the orifice (2).

4. Method according to claim 1, **characterised in that** the lotion (5) is delivered with a velocity of at least 80 m/min.

5. Method according to claim 1, **characterised in that** the lotion (5) is propelled to the surface as a spiral-shaped continuous fiberised string.

6. Method according to claim 1, **characterised in that** the fiberised string has a diameter of less than 2.2 mm.

7. Absorbent article comprising a substantially liquid impermeable back sheet, a substantially liquid permeable topsheet and an absorbent core placed between the back sheet and the topsheet, **characterised in that** a surface of the absorbent article is provided with lotion which has been applied with the method according to claim 1.

8. Absorbent article according to claim 7, **characterised in that** the lotion exhibits a storage modulus of at least 0.2 Pa measured at 55°C.

9. Absorbent article according to claim 8, **characterised in that** the lotion exhibits a storage modulus of at least 0.6 Pa measured at 55°C.

10. Absorbent article according to claim 8, **characterised in that** the lotion exhibits a loss modulus of at least 2.0 Pa measured at 55°C.

11. Absorbent article according to claim 7, **characterised in that** the lotion composition is chosen from:
a) 5% to 50% by weight of a component melting in the range from 25°C to 37°C, chosen from the group of paraffins, fatty acid esters, polyhydroxy fatty acid esters, fatty alcohols, alkoxylated fatty acid esters, alkoxylated fatty alcohols and mixtures of these compounds; and
b) 5% to 50% by weight of a component melting in the range from 40 to 60°C, chosen from the group of polyhydroxy fatty acid esters, C14-C22-fatty alcohols, C12-C22-fatty acids, the alkoxylated derivatives of the fatty alcohols and fatty esters, and mixtures of these components; and
c) 25% to 45% by weight of water.

12. Absorbent article according to claim 7, **characterised in that** the lotion is placed on the topsheet in a continuous fiberised string.

13. Absorbent article according to claim 7, **characterised in that** the diameter of the fiberised string is less than 2.2 mm.

## Patentansprüche

1. Verfahren zum Auftragen einer Lotion (5) auf eine Oberfläche eines absorbierenden Artikels, wobei das Verfahren die Schritte umfasst:
- Platzieren der Oberfläche des absorbierenden Artikels in der Nähe einer Sprühvorrichtung, die einen Sprühkopf (1) umfasst, der eine Öffnung (2) aufweist,
- Drücken der Lotion (5) durch die Sprühkopföffnung (2),
**dadurch gekennzeichnet, dass**
die Lotion (5) auf die Oberfläche als kontinuierlicher faserartiger Strang herausgetrieben wird.

2. Verfahren gemäß Anspruch 1, **dadurch gekennzeichnet, dass** die Oberfläche des absorbierenden Artikels in einem Abstand von 15 bis 300 mm von dem Sprühkopf (1) platziert wird.

3. Verfahren gemäß Anspruch 1, **dadurch gekennzeichnet, dass** die Lotion aus der Öffnung mittels eines Luftstroms (4) herausgetrieben wird, der von der Öffnung (2) getrennt ist.

4. Verfahren gemäß Anspruch 1, **dadurch gekennzeichnet, dass** die Lotion (5) mit einer Geschwindigkeit von mindestens 80 m/min. zugeführt wird.

5. Verfahren gemäß Anspruch 1, **dadurch gekennzeichnet, dass** die Lotion (5) auf die Oberfläche als spiralförmiger, kontinuierlicher, faserartiger Strang herausgetrieben wird.

6. Verfahren gemäß Anspruch 1, **dadurch gekennzeichnet, dass** der faserartige Strang einen Durchmesser von weniger als 2,2 mm aufweist.

7. Absorbierender Artikel, umfassend eine im wesentlichen flüssigkeitsundurchlässige Decklage, eine im wesentlichen flüssigkeitsdurchlässige Innenlage und einen absorbierenden Kern, der zwischen der Decklage und der Innenlage platziert ist, **dadurch gekennzeichnet, dass** eine Oberfläche des absorbierenden Artikels mit Lotion versehen ist, die mit dem Verfahren gemäß Anspruch 1 aufgetragen worden ist.

8. Absorbierender Artikel gemäß Anspruch 7, **dadurch gekennzeichnet, dass** die Lotion ein Speichermodul von mindestens 0,2 Pa, gemessen bei 55°C, aufweist.

9. Absorbierender Artikel gemäß Anspruch 8, **dadurch gekennzeichnet, dass** die Lotion ein Speichermodul von mindestens 0,6 Pa, gemessen bei 55°C, aufweist.

10. Absorbierender Artikel gemäß Anspruch 8, **dadurch gekennzeichnet, dass** die Lotion ein Speichermodul von mindestens 2,0 Pa, gemessen bei 55°C, aufweist.

11. Absorbierender Artikel gemäß Anspruch 7, **dadurch gekennzeichnet, dass** die Lotionszusammensetzung ausgewählt ist aus:
a) 5 bis 50 Gew.% einer Komponente, die im Bereich von 25°C bis 37°C schmilzt, ausgewählt aus der Gruppe von Paraffinen, Fettsäureestern, Polyhydroxyfettsäureestern, Fettalkoholen, alkoxylierten Fettsäureestern, alkoxylierten Fettalkoholen und Mischungen von diesen Verbindungen; und
b) 5 bis 50 Gew.% eine Komponente, die im Bereich von 40 bis 60°C schmilzt, ausgewählt aus der Gruppe von Polyhydroxyfettsäureestern, C14-C22-Fettalkoholen, C12-C22-Fettsäuren, den alkoxylierten Derivaten der Fettalkohole und der Fettester, und Mischungen von diesen Komponenten; und
c) 25 bis 45 Gew.% Wasser.

12. Absorbierender Artikel gemäß Anspruch 7, **dadurch gekennzeichnet, dass** die Lotion auf der Innenlage in einem kontinuierlichen faserartigen Strang platziert ist.

13. Absorbierender Artikel gemäß Anspruch 7, **dadurch gekennzeichnet, dass** der Durchmesser des faserartigen Strangs weniger als 2,2 mm beträgt.

## Revendications

1. Procédé d'application d'une lotion (5) sur une surface d'un article absorbant, lequel procédé comporte les étapes suivantes :
- placer la surface de l'article absorbant à proximité d'un appareil de pulvérisation comprenant une tête de pulvérisation (1), pourvue d'un orifice (2) ;
- et faire passer, en la poussant, la lotion (5) à travers l'orifice (2) de la tête de pulvérisation ;
**caractérisé en ce que** :
la lotion (5) est propulsée vers la surface sous la forme d'un filet continu fibrisé.

2. Procédé conforme à la revendication 1, **caractérisé en ce que** la surface de l'article absorbant est placée à une distance de 15 à 300 mm de la tête de pulvérisation (1).

3. Procédé conforme à la revendication 1, **caractérisé en ce que** la lotion est propulsée au loin de l'orifice par un courant d'air (4) qui ne passe pas par l'orifice (2).

4. Procédé conforme à la revendication 1, **caractérisé en ce que** la lotion (5) est délivrée à une vitesse d'au moins 80 m/min.

5. Procédé conforme à la revendication 1, **caractérisé en ce que** la lotion (5) est propulsée vers la surface sous la forme d'un filet continu fibrisé de forme hélicoïdale.

6. Procédé conforme à la revendication 1, **caractérisé en ce que** le filet fibrisé a moins de 2,2 mm de diamètre.

7. Article absorbant comprenant une feuille de derrière sensiblement imperméable aux liquides, une feuille de dessus sensiblement perméable aux liquides, et un coeur absorbant disposé entre la feuille de derrière et la feuille de dessus, **caractérisé en ce que** la surface de l'article absorbant est pourvue d'une lotion qui y a été appliquée selon un procédé conforme à la revendication 1.

8. Article absorbant conforme à la revendication 7, **caractérisé en ce que** la lotion présente un module de conservation d'au moins 0,2 Pa à 55 °C.

9. Article absorbant conforme à la revendication 8, **caractérisé en ce que** la lotion présente un module de conservation d'au moins 0,6 Pa à 55 °C.

10. Article absorbant conforme à la revendication 8, **caractérisé en ce que** la lotion présente un module de perte d'au moins 2,0 Pa à 55 °C.

11. Article absorbant conforme à la revendication 7, **caractérisé en ce que** la composition de la lotion est choisie dans l'ensemble défini comme suit :
a) de 5 à 50 % en poids d'un composant qui fond entre 25 et 37 °C, choisi dans l'ensemble constitué par les paraffines, esters d'acides gras, esters d'acides gras polyhydroxylés, alcools gras, esters d'acides gras alcoxylés, alcools gras alcoxylés, et mélanges de tels composés ;
b) de 5 à 50 % en poids d'un composant qui fond entre 40 et 60 °C, choisi dans l'ensemble constitué par les esters d'acides gras polyhydroxylés, alcools gras en C₁₄₋₂₂, acides gras en C₁₂₋₂₂, dérivés alcoxylés d'alcools gras ou d'esters gras, et mélanges de tels composés ;
c) et de 25 à 45 % en poids d'eau.

12. Article absorbant conforme à la revendication 7, **caractérisé en ce que** la lotion est disposée sur la feuille de dessus en un filet continu fibrisé.

13. Article absorbant conforme à la revendication 7, **caractérisé en ce que** le filet fibrisé a moins de 2,2 mm de diamètre.
